(19) Europäisches Patentamt
European Patent Office
Office européen des brevets

(11) **EP 3 135 204 A1**

(12) **EUROPEAN PATENT APPLICATION**
published in accordance with Art. 153(4) EPC

(43) Date of publication:
**01.03.2017 Bulletin 2017/09**

(21) Application number: **15783131.4**

(22) Date of filing: **15.04.2015**

(51) Int Cl.:
***A61B 8/08*** (2006.01)

(86) International application number:
**PCT/JP2015/061539**

(87) International publication number:
**WO 2015/163202 (29.10.2015 Gazette 2015/43)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA**

(30) Priority: **21.04.2014 JP 2014087369**

(71) Applicant: **Hitachi, Ltd.**
**Chiyoda-ku,**
**Tokyo 100-8280 (JP)**

(72) Inventors:
• **SONOYAMA, Teruyuki**
**Mitaka-shi**
**Tokyo 181-8622 (JP)**
• **INOUE, Noriaki**
**Mitaka-shi**
**Tokyo 181-8622 (JP)**

(74) Representative: **Strehl Schübel-Hopf & Partner**
**Maximilianstrasse 54**
**80538 München (DE)**

(54) **ULTRASONIC DIAGNOSTIC DEVICE**

(57) A body movement signal generation unit (60) generates a body movement signal which varies in response to body movement of a test subject as a diagnosis subject on the basis of a reception signal corresponding to a monitoring reception beam obtained from a reception unit (14). A body movement monitoring unit (62) determines the start time of a diagnosis-recommended period in which body movement is minimal by distinguishing between large and small body movements on the basis of the body movement signal obtained from the body movement signal generation unit (60). A control unit (70) executes diagnostic processing from a start time for diagnosis. Through this configuration, it is possible to obtain stable diagnostic information in which the effect of heartbeats is low and that is preferably entirely unaffected by heartbeats.

FIG. 1

## Description

### TECHNICAL FIELD

The present invention relates to an ultrasonic diagnostic device, and more particularly to a technology that obtains diagnostic information on a tissue using a shear wave.

### BACKGROUND

In the field of ultrasonic diagnostic devices, there is known a technology which uses a shear wave to obtain diagnostic information on a tissue. In Patent Literature 1, there is described a technology which measures a propagation velocity of a share wave "ShearWave" which is generated in a test subject by a push pulse of an ultrasonic wave and obtains diagnostic information on the elasticity of a tissue from the propagation velocity.

As a technology to obtain diagnostic information related to the elasticity of a tissue, for example, elastography is known to obtain the diagnostic information on the elasticity of the tissue by pressing the tissue in a test subject from the body surface of the test subject and measuring the strain of the tissue generated by the pressure with ultrasonic wave.

### CITATION LIST

### PATENT LITERATURE

Patent Literature 1: JP 2012-100997 A

### SUMMARY

### TECHNICAL PROBLEM

With the elastography for measuring the strain of a tissue, it is difficult to improve measurement accuracy at a part which is rarely pressed manually, such as a liver. Therefore, measurement using a shear wave is normally used as measurement to obtain diagnostic information related to elasticity from, for example, a liver. However, if there is body movement such as, for example, heartbeats or breathing, it is not easy to obtain more stable diagnostic information due to the effect of the body movement in the measurement of, for example, a liver using the shear wave.

In view of the above background technologies, the inventor(s) of this application has carried out repeated research and development into a technology to obtain diagnostic information on a tissue using a shear wave.

The present invention was achieved in the process of this research and development, and its purpose is to improve the accuracy of diagnosis using a shear wave in an ultrasonic diagnostic device.

### SOLUTION TO PROBLEM

A preferable ultrasonic diagnostic device suited to the above object comprises an ultrasonic probe, a transmission unit for controlling the probe to transmit an ultrasonic wave, a reception unit for obtaining a reception signal of the ultrasonic wave received by the probe, a body movement signal generation unit for generating a body movement signal which varies according to a body movement of a test subject based on a reception signal of the ultrasonic wave related to the test subject, and a body movement monitoring unit for determining start time of a diagnosis-recommended period in which the body movement is small by discriminating between large and small body movements based on the body movement signal, wherein diagnosis processing is started from the start time, and a shear wave is generated in the test subject by the diagnosis processing to obtain diagnostic information on a tissue in the test subject.

According to the above device, it becomes possible to obtain stable diagnostic information without much influence due to the body movement, and desirably without any influence due to the body movement because the diagnosis processing is started from the start time of the diagnosis-recommended period when the body movement is small.

According to a desired specific example, the ultrasonic diagnostic device generates a body movement signal based on a reception signal obtained by transmitting a monitoring ultrasonic wave, determines the start time by discriminating between large and small body movements based on the body movement signal, generates a shear wave in the test subject by transmitting a pushing ultrasonic wave from the start time, and obtains diagnostic information on the tissue by measuring a displacement of the tissue in the test subject accompanying the shear wave based on the reception signal obtained by transmitting a tracking ultrasonic wave.

According to a desired specific example, the body movement monitoring unit detects a feature wave contained in the body movement signal by discriminating between large and small body movements based on the body movement signal to determine the start time according to timing for detecting the feature wave.

According to a desired specific example, the body movement monitoring unit detects the feature wave corresponding to a time phase in which the body movement accompanying heartbeats becomes maximum to determine as the start time a time delayed by a start delay time from the feature wave detection time.

According to a desired specific example, the body movement monitoring unit discriminates between large and small body movements based on the body movement signal to determine as the start timing a time delayed by a start waiting time from a time of a diagnostic start operation by a user in a state where the body movement is small.

ADVANTAGEOUS EFFECTS OF INVENTION

The accuracy of diagnosis using a shear wave in the ultrasonic diagnostic device is improved by the present invention. For example, according to a preferable embodiment of the invention, it becomes possible to obtain stable diagnostic information without much influence due to the body movement, and desirably without any influence due to the body movement because the diagnosis processing is started from the start time of the diagnosis-recommended period when the body movement is small.

BRIEF DESCRIPTION OF DRAWINGS

Embodiment(s) of the present disclosure will be described based on the following figures, wherein:

FIG. 1 is a diagram showing the overall structure of a preferable ultrasonic diagnostic device in implementation of the invention;
FIG. 2 is a diagram illustrating a specific example of monitoring processing based on a body movement signal;
FIG. 3 is a diagram illustrating a difference in amplitude between time phases of a reception signal;
FIG. 4 is a diagram illustrating specific examples of diagnosis start time; and
FIG. 5 is a diagram illustrating a specific example of diagnosis processing using a shear wave.

DESCRIPTION OF EMBODIMENTS

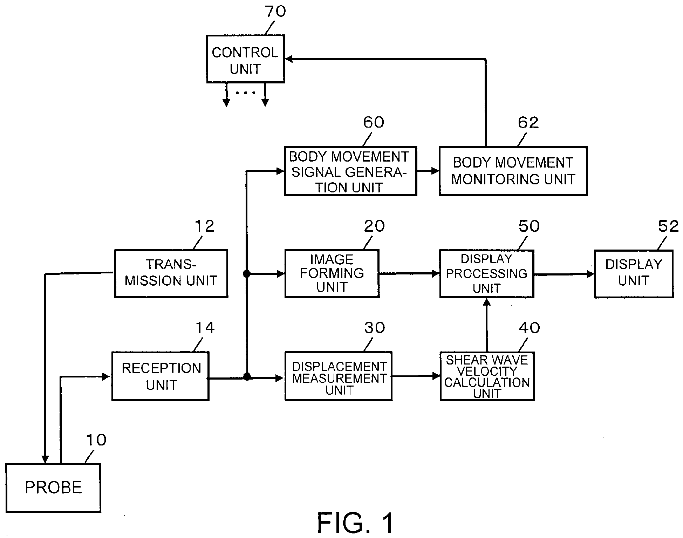

FIG. 1 is a diagram showing the overall structure of a preferable ultrasonic diagnostic device in implementation of the invention. A probe 10 is an ultrasonic probe which sends/receives an ultrasonic wave to/from a tissue in a test subject, for example, an area including a liver or the like in a living body. The probe 10 has plural vibration elements which individually send/receive or transmit an ultrasonic wave, and the plural vibration elements are controlled for transmission by a transmission unit 12 to form a transmission beam.

Also, the plural vibration elements equipped by the probe 10 receive the ultrasonic wave from the area including the tissue of the liver or the like, the signal thus obtained is output to a reception unit 14, and the reception unit 14 forms a reception beam to obtain a reception signal (echo data) along the reception beam.

The probe 10 has a function to transmit an ultrasonic wave (push pulse) for generating a shear wave in an area including tissue of a liver or the like in a test subject, a function to send/receive an ultrasonic wave (tracking pulse) for measuring a displacement of the tissue accompanying the shear wave, and a function to send/receive an image forming ultrasonic wave.

The probe 10 also has a function to transmit a monitoring ultrasonic wave for monitoring the body movement in the test subject. The image forming ultrasonic wave may also be used partly or wholly as the monitoring ultrasonic wave.

Transmission of the ultrasonic wave is controlled by the transmission unit 12. When the shear wave is generated, the transmission unit 12 outputs a push pulse transmission signal to the plural vibration elements which are equipped in the probe 10, thereby forming a push pulse transmission beam. Also, when the shear wave is measured, the transmission unit 12 outputs a tracking pulse transmission signal to the plural vibration elements which are equipped in the probe 10, thereby forming a tracking pulse transmission beam.

Further, when an ultrasonic image is formed, the transmission unit 12 outputs an image forming transmission signal to the plural vibration elements which are equipped in the probe 10, and the image forming transmission beam is scanned. Also, when the body movement within the test subject is monitored, the transmission unit 12 outputs a monitoring transmission signal to the plural vibration elements which are equipped in the probe 10, thereby forming a monitoring transmission beam.

Based on a received wave signal obtained from the plural vibration elements when the probe 10 sends/receives the tracking pulse, the reception unit 14 forms the reception beam of the tracking pulse and obtains a reception signal corresponding to the reception beam. Also, based on the received wave signal obtained from the plural vibration elements when the probe 10 sends/receives an image forming ultrasonic wave, the reception unit 14 forms an image forming reception beam and generates a reception signal corresponding to the reception beam. In addition, based on the received wave signal obtained from the plural vibration elements when the probe 10 sends/receives a monitoring ultrasonic wave, the reception unit 14 also forms a monitoring reception beam and generates a reception signal corresponding to the reception beam.

The image forming ultrasonic beam (transmission beam and reception beam) is scanned in a two-dimensional plane including tissue of a liver or the like which is to be a diagnosis subject, and image forming reception signals are collected from the two-dimensional plane. The image forming ultrasonic beam may naturally be scanned three-dimensionally in a three-dimensional space to collect the image forming reception signals from the three-dimensional space.

An image forming unit 20 forms image data of the ultrasonic wave based on the image forming reception signal collected by the reception unit 14. The image forming unit 20 forms, for example, image data of a B-mode image (tomographic image) of an area including tissue of a liver or the like which is a diagnosis subject. Also, when the image forming reception signals are being collected three-dimensionally, the image forming unit 20 may form image data of a three-dimensional ultrasonic image.

A displacement measurement unit 30 generates displacement data indicating a displacement of the shear wave over plural time phases based on the reception signal corresponding to the reception beam of the tracking pulse obtained from the reception unit 14. Also, a shear wave velocity calculation unit 40 calculates a velocity of the shear wave based on the displacement data which is obtained from the displacement measurement unit 30. Processing by the displacement measurement unit 30 and the shear wave velocity calculation unit 40 is described later in detail.

A display processing unit 50 forms a display image based on the image data of the ultrasonic image obtained from the image forming unit 20 and the velocity of the shear wave calculated by the shear wave velocity calculation unit 40. The display image formed by the display processing unit 50 is displayed on a display unit 52.

A body movement signal generation unit 60 generates a body movement signal which varies according to the body movement of the test subject as a diagnosis subject based on the reception signal corresponding to the monitoring reception beam obtained from the reception unit 14. Also, a body movement monitoring unit 62 discriminates between large and small body movements based on the body movement signal obtained from the body movement signal generation unit 60 to determine start time of a diagnosis-recommended period in which the body movement is small. Processing by the body movement signal generation unit 60 and the body movement monitoring unit 62 is described later in detail.

A control unit 70 performs overall control of the inside of the ultrasonic diagnostic device shown in FIG. 1. Through the control, the control unit 70 starts the diagnosis processing according to the result of monitoring the body movement by the body movement monitoring unit 62.

Among the individual structures (individual function blocks) shown in FIG. 1, the transmission unit 12, the reception unit 14, the image forming unit 20, the displacement measurement unit 30, the shear wave velocity calculation unit 40, the display processing unit 50, the body movement signal generation unit 60, and the body movement monitoring unit 62 can each be realized using, for example, hardware such as an electric/electronic circuit and a processor, and if necessary, a device such as a memory may be used for the realization. Also, a preferable specific example of the display unit 52 is a liquid crystal display or the like. The control unit 70 can also be realized by, for example, cooperation between hardware such as a CPU or a processor and a memory, and software (program) which regulates the operation of the CPU or the processor.

The overview of the ultrasonic diagnostic device of FIG. 1 is as described above. Next, the body movement monitoring processing and the tissue diagnosis processing by the ultrasonic diagnostic device of FIG. 1 are described in detail. Also, for the individual structures (individual function blocks) shown in FIG. 1, reference numerals in FIG. 1 are used in the following description.

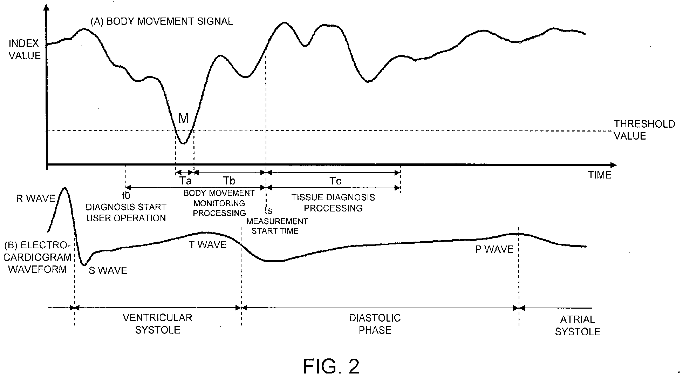

FIG. 2 is a diagram illustrating a specific example of monitoring processing based on a body movement signal. FIG. 2(A) illustrates a preferable specific example of the body movement signal related to a test subject as a diagnosis subject. Also, as reference information for illustrating the body movement signal of FIG. 2(A), FIG. 2(B) illustrates a specific example of an electrocardiogram waveform which is obtained from the same test subject by using an electrocardiograph or the like. The electrocardiogram waveform includes plural feature waves (R wave, S wave, T wave, and P wave). The R wave is a waveform part having the largest amplitude in the electrocardiogram waveform, and is generally generated once in a period of heartbeats. The S wave is generated immediately after the R wave, and then the T wave and the P wave are generated. Also, the body movement signal of FIG. 2(A) and the electrocardiogram waveform of FIG. 2(B) are waveforms on the same time axis.

For example, when the operation to start diagnosis is received at time t0 from a user via an operation device such as an operation panel, the control unit 70 starts the control related to body movement monitoring processing, and a monitoring ultrasonic wave is transmitted to the test subject including a liver or the like, which is a diagnosis subject, to obtain a monitoring reception signal.

The body movement signal generation unit 60 generates the body movement signal shown in, for example, FIG. 2(A) based on the reception signal corresponding to the monitoring reception beam. The body movement signal generation unit 60 generates a body movement signal based on a difference of amplitude between, for example, time phases of the reception signal based on the reception signal of the monitoring reception beam passing through a liver or the like as a diagnosis subject.

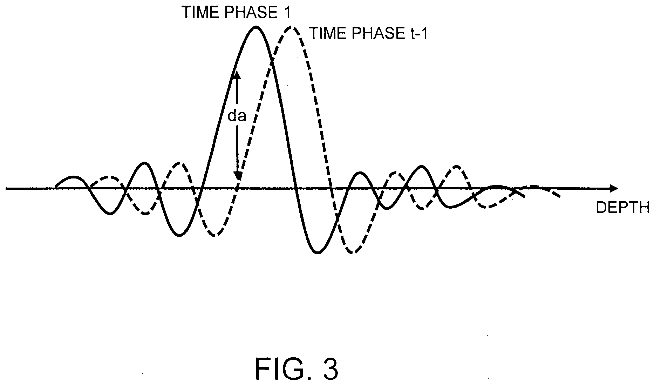

FIG. 3 is a diagram illustrating a difference in amplitude between time phases of a reception signal. FIG. 3 illustrates a waveform (solid line) of the reception signal in a time phase t, and a waveform (broken line) of the reception signal in a time phase t-1 which is earlier by one time phase than the time phase t. Also, the one time phase in FIG. 3 is, for example, one period of a pulse repeating time (PRT) of the monitoring reception beam. Incidentally, when the monitoring reception beam is being scanned in the two-dimensional plane, one frame becomes one time phase.

The body movement signal generation unit 60 calculates a difference in amplitude da between time phases for the reception signal of the time phase t and the reception signal of the time phase t-1 related to the monitoring reception beam. The difference da may be calculated from the amplitude value at a specified point (specified depth) or may be calculated from the amplitude value at plural points (plural depths) by, for example, statistical operation (such as an average operation). Also, when the monitoring reception beam is being scanned in the two-dimensional plane including the diagnosis subject such as a liver, the difference da may be calculated by the statistical operation in the plane based on the reception signal obtained in the plane (in a cross section).

The body movement signal generation unit 60 calculates the difference da (FIG. 3) for each time phase t and generates a body movement signal (FIG. 2) indicating an index value which varies over plural time phases. The difference da which is obtained for each time phase t has a smaller value for a smaller body movement, and has a larger value for a larger body movement. The body movement signal generation unit 60 turns the waveform of the difference da obtained over the plural time phases vertically (longitudinal axis direction) to generate the body movement signal (FIG. 2). Thus, the body movement signal (FIG. 2) is generated which has a larger index value with smaller body movement and has a smaller index value with larger body movement.

In addition, the body movement signal generation unit 60 may form the body movement signal (FIG. 2) with the correlation value of each time phase t as an index value by calculating the correlation value of each time phase t using expression MATH. 1 based on a reception signal of the monitoring reception beam, for example, a complex reception signal after orthogonal detection processing.

[MATH. 1]

$$R_d(t) = \sum_{t=-T}^{T} IQ_d(t)\overline{IQ_d(t-1)}$$

R: correlation value
IQ: complex reception signal
d: sample in depth direction
T: range in time direction to perform correlation processing

For example, one monitoring ultrasonic beam is formed to pass through a tissue of a liver or the like or to pass near the tissue of the liver or the like, and a correlation value of each time phase t is calculated by the expression MATH. 1 based on a reception signal which is obtained from the one ultrasonic beam. Also, there may be only 1 sample d in a depth direction in the expression MATH. 1 may be one, but there may also be plural samples for sample d in the depth direction, and the correlation value obtained by the expression MATH. 1 may be added to the depth direction to improve the sensitivity of the correlation value.

For example, a waveform of the correlation value may be generated by scanning the monitoring ultrasonic beam (transmission beam and reception beam) in a plane including a tissue of a liver or the like to form a monitoring frame, sequentially forming plural monitoring frames over plural time phases, and calculating a correlation value for each time phase from the plural monitoring frames.

Also, the body movement signal generation unit 60 may generate a body movement signal, which has Doppler information variable over the plural time phases, as an index value, based on the Doppler information (e.g., Doppler shift frequency) which is obtained for each time phase through the monitoring ultrasonic beam.

Returning to FIG. 2, the body movement monitoring unit 62 discriminates between large and small body movements based on the body movement signal, to determine start time of the diagnosis-recommended period in which the body movement is small. The body movement monitoring unit 62 detects a feature wave M corresponding to the time phase in which the body movement accompanying heartbeats becomes maximum, based on the body movement signal generated due to the body movement signal generation unit 60, for example a waveform of the index value over plural time phases shown in FIG. 2(A), and determines, as diagnosis start time, a time ts delayed by a start delay time Tb from the detection time of the feature wave M.

In the expansion and contraction movement of the heart, there is a time phase having the largest change during a ventricular systole when the ventricle contracts, and the effect of the body movement due to heartbeats becomes largest in the period corresponding to this time phase. Also, when the effect of the body movement due to heartbeats is strong, the difference da (FIG. 3) becomes large, and an index value of the body movement signal (FIG. 2) obtained by turning the waveform of the difference da vertically (longitudinal axis direction) becomes small.

Then, the body movement monitoring unit 62 retrieves, for example, a waveform part where the index value of the body movement signal becomes a threshold value or below to detect the feature wave M. Specifically, when a waveform part with the threshold value or below continues for a detection period Ta (e.g., 10 ms to 150 ms), its waveform part is detected as the feature wave M.

Further, the body movement monitoring unit 62 determines, as diagnosis start time, a time ts elapsed by a start delay time Tb (e.g., about 100 ms) from the detection timing of the feature wave M. Also, the control unit 70 starts the control related to diagnosis processing of a tissue from the diagnosis start time, thereby executing the diagnosis processing of a tissue of a liver or the like using a shear wave. Incidentally, the monitoring processing of the body movement may also be executed after the diagnosis processing of the tissue has been executed for a designated diagnosis time Tc.

In addition, in the specific example shown in FIG. 2, a level (magnitude) of the threshold value, a time length of the detection period Ta, a time length of the start delay time Tb, and a time length of the diagnosis time Tc each may be predetermined values (default value) or may be adjusted appropriately by a user such as a doctor.

Also, FIG. 2(A) shows a specific example of a body movement signal which is obtained by vertical (longitudinal axis direction) turning of a waveform of the difference da (FIG. 3) which is obtained over the plural time phases, but the waveform of the difference da may be used as it is (without turning) as the body movement signal. When the waveform of the difference da is used as it is, the index value (difference da) of the body movement signal becomes large if the effect of the body movement due to heartbeats is strong, so that a waveform part where the index value becomes the threshold value or more may be retrieved to detect the feature wave M.

In addition, when a waveform corresponding to the electrocardiogram waveform shown in, for example, FIG. 2(B) is obtained on the basis of the reception signal of the monitoring reception beam, a time phase corresponding to the R wave or the T wave in the electrocardiogram waveform is detected, and a time ts delayed by a start delay time Tb (start delay time Tb adjusted for the electrocardiogram waveform) from the time phase may be determined as diagnosis start time.

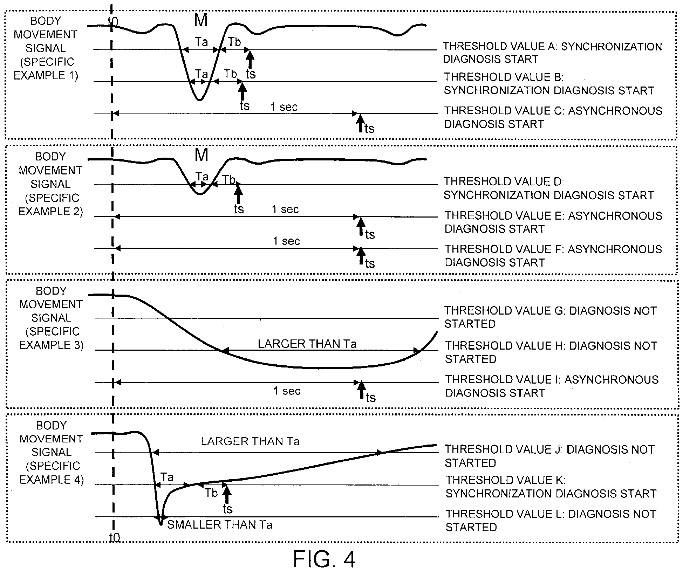

FIG. 4 is a diagram illustrating specific examples of diagnosis start time. FIG. 4 illustrates various specific examples of waveforms and threshold values of the body movement signals (FIG. 2(A)).

When the waveform of the body movement signal shown in a specific example 1 is obtained and a threshold value A (or a threshold value B) is set, a waveform part with the threshold value A (or the threshold value B) or below continues for the detection period Ta (e.g., 10 ms to 150 ms), so that the waveform part is detected as the feature wave M, and a time ts delayed by a start delay time Tb (e.g., 100 ms) from the detection time of the feature wave M is determined as diagnosis start time. Then, diagnosis is started in synchronization with the time of the feature wave M.

On the other hand, when a threshold value C is set in the specific example 1, a waveform part with the threshold value C or below is not detected. In this case, the body movement signal continuously exceeds the threshold value C, it is judged that the effect due to the body movement due to heartbeats or the like is small, and a time ts delayed by a start waiting time (e.g., 1 sec) from a time t0 when the diagnostic start operation is received from a user is determined as diagnosis start time. Then, asynchronous diagnosis not in synchronization with the feature wave M is started.

Also, when the waveform of the body movement signal shown in a specific example 2 is obtained and a threshold value D is set, a waveform part with the threshold value D or below continues for the detection period Ta (e.g., 10 ms to 150 ms), so that the waveform part is detected as the feature wave M, and a time ts elapsed by a start delay time Tb (e.g., 100 ms) from the detection time of the feature wave M is determined as diagnosis start time. Then, diagnosis is started in synchronization with the time of the feature wave M.

On the other hand, when a threshold value E (or a threshold value F) is set in the specific example 2, a waveform part with the threshold value E (or the threshold value F) or below is not detected. In this case, the body movement signal continuously exceeds the threshold value E (or the threshold value F), and it is judged that the effect due to the body movement such as heartbeats is small, a time ts delayed by a start waiting time (e.g., 1 sec.) from a time t0 when a diagnostic start operation is received from a user is determined as diagnosis start time. Then, asynchronous diagnosis not in synchronization with the feature wave M is started.

Also, when a waveform of the body movement signal shown in a specific example 3 is obtained and a threshold value G (or a threshold value H) is set, a waveform part with the threshold value G (or the threshold value H) or below continues for longer than the detection period Ta (e.g., 10 ms to 150 ms) (e.g., longer than 150 ms), so that the waveform part is not detected as the feature wave M. In this case, since the correlation value is continuously smaller than the threshold value G (or the threshold value H), it is judged that the effect due to the body movement is large, and diagnosis is not started. Also, in this case, it is desirable that a user is informed by showing on the display unit 52 that the diagnosis cannot be started because the body movement is large.

On the other hand, when a threshold value I is set in the specific example 3, a waveform part with the threshold value I or below is not detected. In this case, the body movement signal continuously exceeds the threshold value I, it is judged that the effect due to the body movement such as heartbeats is small, and a time ts delayed by a start waiting time (e.g., 1 sec) from a time t0 when the diagnostic start operation was received from the user is determined as diagnosis start time. Then, asynchronous diagnosis is started without synchronization with the feature wave M.

In addition, as shown in a specific example 4, when a waveform of the body movement signal is obtained and a threshold value J is set, a waveform part with the threshold value J or below continues longer than the detection period Ta (e.g., 10 ms to 150 ms) (e.g., longer than 150 ms), so that the waveform part is not detected as the feature wave M. In this case, it is judged that the effect due to the body movement is large, and diagnosis is not started. Also, a user may be informed by showing on the display unit 52 that diagnosis cannot be started because the body movement is large.

On the other hand, when a threshold value K is set in the specific example 4, a waveform part with the threshold value K or below continues for the detection period Ta (e.g., 10 ms to 150 ms), the waveform part is detected as the feature wave M, and a time ts delayed by a start delay time Tb (e.g., 100 ms) from detection time of the feature wave M is determined as diagnosis start time. Then, diagnosis is started in synchronization with timing of the feature wave M.

Also, when a threshold value L is set in the specific example 4, a waveform part with the threshold value L or below is smaller (shorter) than the detection period Ta (e.g., 10 ms to 150 ms), the waveform part is not detected as the feature wave M. In this case, it is judged that the body movement might be changed largely due to an effect other than heartbeats, and diagnosis is not started. In this case, it is also desirable that a user is informed by showing on the display unit 52 that diagnosis cannot be started.

In addition, the waveform of the body movement signal shown in the specific example 4 might be affected due to the body movement other than heartbeats or due to noise or the like. Then, it may be determined whether diagnosis is executed by comprehensive determination based on, for example, plural threshold values (e.g., threshold values J, K, and L).

When diagnosis start time is determined by monitoring processing of the body movement based on the body movement signal, the control unit 70 starts the control related to diagnosis processing of a tissue from the diagnosis start time. Then, the diagnosis processing of a tissue of a liver or the like using the shear wave is executed.

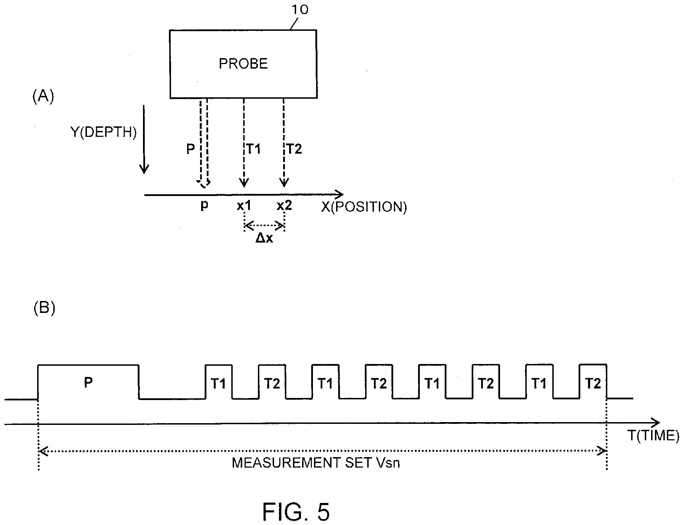

FIG. 5 is a diagram illustrating a specific example of the diagnosis processing using the shear wave. FIG. 5(A) illustrates a specific example of a transmission beam P of a push pulse and ultrasonic beams T1, T2 of a tracking pulse formed by using the probe 10.

In FIG. 5(A), the transmission beam P of the push pulse is formed along the depth Y direction to pass through a position p in the X direction. For example, the transmission beam P of the push pulse is formed with the position p on the X-axis shown in FIG. 5(A) as a focal point. For example, the position p is set as a desired position by a user (tester) such as a doctor who has confirmed an ultrasonic image on a diagnosis subject such as a liver within a living body shown on the display unit 52.

When the transmission beam P of the push pulse is formed with the position p used as the focal point and the push pulse is transmitted, a relatively strong shear wave is generated with the position p used as a starting point in the living body. In the specific example shown in FIG. 5(A), a propagation velocity in the X direction of the shear wave which is generated with the position p as a center is measured.

In FIG. 5(A), two ultrasonic beams T1, T2 related to the tracking pulse are formed. The ultrasonic beam (transmission beam and reception beam) T1 is formed to pass through a position x1 on the X-axis shown in, for example, FIG. 5(A), and the ultrasonic beam (transmission beam and reception beam) T2 is formed to pass through a position x2 on the X-axis shown in, for example, FIG. 5(A). The position x1 and the position x2 may be set, for example, at desired positions by a user who has confirmed the ultrasonic image of a liver or the like displayed on the display unit 52, and the ultrasonic diagnostic device of FIG. 1 may set the position x1 and the position x2 at points away from the position p by a prescribed distance along the X direction.

FIG. 5(B) shows a specific example of generation timing of the transmission beam P of a push pulse and ultrasonic beams T1, T2 of a tracking pulse. The horizontal axis in FIG. 5(B) is a time axis t.

In FIG. 5(B), the period P is a period in which a transmission beam P of a push pulse is formed, and periods T1, T2 each are periods in which ultrasonic beams T1, T2 of a tracking pulse are formed.

In the period P, a push pulse of multiple waves is transmitted. For example, an ultrasonic wave of a continuous wave is transmitted in the period P. Thus, a shear wave is generated at, for example, the position p.

In the periods T1, T2, a so-called tracking pulse of pulse waves of approximately one to several waves is transmitted, and a reflected wave accompanying the pulse wave is received. For example, the ultrasonic beams T1, T2 passing through the positions x1, x2 are formed, and reception signals at the positions x1, x2 are obtained.

The tracking pulse is sent/received repeatedly over the plural periods. That is to say, as shown in FIG. 5(B), the periods T1, T2 are alternately repeated until, for example, a displacement of a tissue accompanying the shear wave is confirmed.

The displacement measurement unit 30 measures a displacement at the positions x1, x2 based on the received data of the ultrasonic beam T1 and the received data of the ultrasonic beam T2 of the tracking pulse.

The shear wave velocity calculation unit 40 calculates, for example, a propagation velocity $Vs=\Delta x/(t2-t1)$ in the X-axis direction of the shear wave based on a time t1 when a displacement of a tissue at a position x1 becomes maximum, a time t2 when a displacement of a tissue at a position x2 becomes maximum, and a distance $\Delta x$ between the position x1 and the position x2 due to the effect of the shear wave generated at the position p. Incidentally, the propagation velocity of the shear wave may be calculated by another known technique. Further, based on the propagation velocity of the shear wave, the elasticity value or the like of the tissue with the shear wave measured may be calculated.

The measurement set Vsn shown in FIG. 5(B) is a period from the start of the push pulse transmission to the calculation of the propagation velocity of the shear wave. The control unit 70 may execute, for example, the diagnosis processing from the diagnosis start time to execute the measurement set Vsn for one set and to return to the monitoring processing of the body movement. Also, the control unit 70 may execute, for example, diagnosis processing from the diagnosis start time to execute plural measurement sets Vsn during the predesignated diagnosis time Tc (see FIG. 2) and to return to the monitoring processing of the body movement.

In addition, in the specific example of FIG. 5, the ultrasonic beams T1, T2 of the tracking pulse are formed on the positive directional side of the X-axis with respect to the transmission beam P of the push pulse, but it may be configured so that the ultrasonic beams T1, T2 of the tracking pulse are formed on the negative directional side of the X-axis with respect to the transmission beam P of the push pulse, and the shear wave propagating to the negative directional side of the X-axis is measured. It is naturally desirable that the position p of the transmission beam P of the push pulse and the positions x1, x2 of the ultrasonic beams T1, T2 of the tracking pulse are appropriately set according to a diagnosis subject and diagnostic conditions.

Thus, according to the ultrasonic diagnostic device of FIG. 1, the diagnosis processing is started from the start time of the diagnosis-recommended period in which the body movement is small, so that it becomes possible to obtain stable diagnosis information with less effect due to the body movement, and desirably without any effect of the body movement, to obtain, for example, the propagation velocity Vs of the shear wave.

In addition, when the propagation velocity Vs is calculated by the shear wave velocity calculation unit 40, the display processing unit 50 forms a display image including the propagation velocity Vs, and the display image is shown on the display unit 52. Also, together with the propagation velocity Vs or instead of the propagation velocity Vs, diagnostic information related to tissue hardness may be calculated and displayed based on the propagation velocity Vs. For example, as the diagnostic information related to the hardness, Young's modulus $E=3pVs2$ (p: density) may be calculated based on the propagation velocity Vs and displayed.

While preferable embodiments of the present invention have been described, the above-described embodiments are mere examples in all respects and do not limit the scope of the invention. The invention includes various types of modified embodiments without departing from the essence of the invention.

REFERENCE SIGNS LIST

10: Probe, 12: Transmission unit, 14: Reception unit, 20: Image forming unit, 30: Displacement measurement unit, 40: Shear wave velocity calculation unit, 50: Display processing unit, 52: Display unit, 60: Body movement signal generation unit, 62: Body movement monitoring unit, 70: Control unit.

**Claims**

1. An ultrasonic diagnostic device, comprising:

    an ultrasonic probe,
    a transmission unit for controlling the probe to transmit an ultrasonic wave,
    a reception unit for obtaining a reception signal of the ultrasonic wave received by the probe,
    a body movement signal generation unit for generating a body movement signal which varies according to a body movement of a test subject based on a reception signal of the ultrasonic wave related to the test subject, and
    a body movement monitoring unit for determining start time of a diagnosis-recommended period in which the body movement is small by discriminating between large and small body movements based on the body movement signal, wherein:

       diagnosis processing is started from the start time, and a shear wave is generated in the test subject by the diagnosis processing to obtain diagnostic information on a tissue in the test subject.

2. The ultrasonic diagnostic device according to Claim 1, wherein:

       the body movement signal is generated based on the reception signal obtained by transmitting a monitoring ultrasonic wave, and the start time is determined by discriminating between large and small body movements based on the body movement signal.

3. The ultrasonic diagnostic device according to Claim 2, wherein:

       a shear wave is generated in the test subject by transmitting a pushing ultrasonic wave from the start time, and diagnostic information on the tissue is obtained by measuring a displacement of a tissue in the test subject accompanying the shear wave based on the reception signal obtained by transmitting a tracking ultrasonic wave.

4. The ultrasonic diagnostic device according to Claim 1, wherein:

       the body movement monitoring unit detects a feature wave contained in the body movement signal by discriminating between large and small body movements based on the body movement signal to determine the start time according to time for detecting the feature wave.

5. The ultrasonic diagnostic device according to Claim 3, wherein:

       the body movement monitoring unit detects a feature wave contained in the body movement signal by discriminating between large and small body movements based on the body movement signal to determine the start time according to timing for detecting the feature wave.

6. The ultrasonic diagnostic device according to Claim 4, wherein:

the body movement monitoring unit detects the feature wave corresponding to a time phase in which the body movement accompanying heartbeats becomes maximum to determine as the start time a time delayed by a start delay time from the feature wave detection timie

7. The ultrasonic diagnostic device according to Claim 5, wherein:

the body movement monitoring unit detects the feature wave corresponding to a time phase in which the body movement accompanying heartbeats becomes maximum to determine as the start time a time delayed by a start delay time from the feature wave detection time.

8. The ultrasonic diagnostic device according to Claim 1, wherein:

the body movement monitoring unit discriminates between large and small body movements based on the body movement signal to determine as the start time a time delayed by a start waiting time from time of a diagnostic start operation by a user in a state where the body movement is small.

9. The ultrasonic diagnostic device according to Claim 3, wherein:

the body movement monitoring unit discriminates between large and small body movements based on the body movement signal to determine as the start time a time delayed by a start waiting time from time of a diagnostic start operation by a user in a state where the body movement is small.

10. The ultrasonic diagnostic device according to Claim 5, wherein:

the body movement monitoring unit discriminates between large and small of the body movement based on the body movement signal to determine as the start time a time delayed by a start waiting time from time of a diagnostic start operation by a user in a state where the body movement is small.

11. The ultrasonic diagnostic device according to Claim 1, wherein:

the body movement monitoring unit, when a feature wave contained in the body movement signal and corresponding to a time phase in which a body movement accompanying heartbeats becomes maximum can be detected by monitoring processing executed after receiving a diagnostic start operation by a user, determines as the start time a time delayed by a start delay time from the feature wave detection time, and when the feature wave can not be detected, determines as the start time a time delayed by a start waiting time in a state where the body movement is small from the time of the diagnostic start operation.

12. The ultrasonic diagnostic device according to Claim 3, wherein:

the body movement monitoring unit, when a feature wave contained in the body movement signal and corresponding to a time phase in which a body movement accompanying heartbeats becomes maximum can be detected by monitoring processing executed after receiving a diagnostic start operation by a user, determines as the start time a time delayed by a start delay time from the feature wave detection time, and when the feature wave can not be detected, determines as the start time a time delayed by a start waiting time in a state where the body movement is small from the time of the diagnostic start operation.

70

CONTROL
UNIT

· · ·

60

BODY MOVEMENT
SIGNAL GENERA-
TION UNIT

62

BODY MOVEMENT
MONITORING UNIT

12

TRANS-
MISSION UNIT

20

IMAGE
FORMING
UNIT

50

DISPLAY
PROCESSING
UNIT

52

DISPLAY
UNIT

14

RECEPTION
UNIT

30

DISPLACEMENT
MEASUREMENT
UNIT

40

SHEAR WAVE
VELOCITY
CALCULATION
UNIT

10

PROBE

FIG. 1

10

FIG. 2

FIG. 3

BODY MOVEMENT SIGNAL (SPECIFIC EXAMPLE 1)

M

Ta   Tb

Ta   Tb   ts

ts   1 sec

ts

THRESHOLD VALUE A: SYNCHRONIZATION DIAGNOSIS START

THRESHOLD VALUE B: SYNCHRONIZATION DIAGNOSIS START

THRESHOLD VALUE C: ASYNCHRONOUS DIAGNOSIS START

BODY MOVEMENT SIGNAL (SPECIFIC EXAMPLE 2)

M

Ta   Tb

ts   1 sec

ts

1 sec

ts

THRESHOLD VALUE D: SYNCHRONIZATION DIAGNOSIS START

THRESHOLD VALUE E: ASYNCHRONOUS DIAGNOSIS START

THRESHOLD VALUE F: ASYNCHRONOUS DIAGNOSIS START

BODY MOVEMENT SIGNAL (SPECIFIC EXAMPLE 3)

LARGER THAN Ta

1 sec   ts

THRESHOLD VALUE G: DIAGNOSIS NOT STARTED

THRESHOLD VALUE H: DIAGNOSIS NOT STARTED

THRESHOLD VALUE I: ASYNCHRONOUS DIAGNOSIS START

BODY MOVEMENT SIGNAL (SPECIFIC EXAMPLE 4)

LARGER THAN Ta

Ta

Tb

ts

SMALLER THAN Ta

t0

THRESHOLD VALUE J: DIAGNOSIS NOT STARTED

THRESHOLD VALUE K: SYNCHRONIZATION DIAGNOSIS START

THRESHOLD VALUE L: DIAGNOSIS NOT STARTED

t0

FIG. 4

FIG. 5

## INTERNATIONAL SEARCH REPORT

| | International application No. |
|---|---|
| | PCT/JP2015/061539 |

**A.    CLASSIFICATION OF SUBJECT MATTER**
*A61B8/08*(2006.01)i

According to International Patent Classification (IPC) or to both national classification and IPC

**B.    FIELDS SEARCHED**

Minimum documentation searched (classification system followed by classification symbols)
A61B8/00-8/15

Documentation searched other than minimum documentation to the extent that such documents are included in the fields searched
Jitsuyo Shinan Koho          1922-1996    Jitsuyo Shinan Toroku Koho    1996-2015
Kokai Jitsuyo Shinan Koho    1971-2015    Toroku Jitsuyo Shinan Koho    1994-2015

Electronic data base consulted during the international search (name of data base and, where practicable, search terms used)

**C.    DOCUMENTS CONSIDERED TO BE RELEVANT**

| Category* | Citation of document, with indication, where appropriate, of the relevant passages | Relevant to claim No. |
|---|---|---|
| Y<br>A | JP 2013-500752 A  (Super Sonic Imagine),<br>10 January 2013 (10.01.2013),<br>paragraphs [0045], [0078]; fig. 1<br>& US 2011/0028838 A1     & WO 2011/012340 A1<br>& EP 2535004 A1          & CA 2769253 A1<br>& KR 10-2012-0071385 A   & CN 102724917 A | 1-5<br>6-12 |
| Y<br>A | JP 2012-528614 A  (Super Sonic Imagine),<br>15 November 2012 (15.11.2012),<br>paragraphs [0050], [0056] to [0061]; fig. 1 to 2<br>& US 2010/0312116 A1     & WO 2010/139519 A1<br>& CA 2764263 A1          & CN 102458260 A | 1-5<br>6-12 |

| ☒    Further documents are listed in the continuation of Box C. | ☐    See patent family annex. |
|---|---|

| * Special categories of cited documents: | "T" later document published after the international filing date or priority date and not in conflict with the application but cited to understand the principle or theory underlying the invention |
|---|---|
| "A" document defining the general state of the art which is not considered    to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
|---|---|
| 03 July 2015 (03.07.15) | 14 July 2015 (14.07.15) |

| Name and mailing address of the ISA/<br>    Japan Patent Office<br>    3-4-3,Kasumigaseki,Chiyoda-ku,<br>    Tokyo 100-8915,Japan | Authorized officer<br><br>Telephone No. |
|---|---|

Form PCT/ISA/210 (second sheet) (July 2009)

**INTERNATIONAL SEARCH REPORT**

| International application No. |
| --- |
| PCT/JP2015/061539 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y<br>A | JP 10-99328 A (Toshiba Corp.),<br>21 April 1998 (21.04.1998),<br>paragraphs [0040], [0045] to [0047]<br>& US 2001/0024516 A1 | 1-5<br>6-12 |
| E,X | JP 2015-107311 A (GE Medical Systems Global Technology Co., L.L.C.),<br>11 June 2015 (11.06.2015),<br>entire text; all drawings<br>(Family: none) | 1-5,8-10 |
| A | JP 2013-544615 A (Koninklijke Philips N.V.),<br>19 December 2013 (19.12.2013),<br>paragraphs [0021] to [0022]<br>& US 2013/0296698 A1    & WO 2012/080913 A1 | 1-12 |
| A | JP 2012-100997 A (Hitachi Medical Corp.),<br>31 May 2012 (31.05.2012),<br>paragraph [0033]<br>(Family: none) | 1-12 |
| A | JP 2002-538911 A (Societe d'Elastographie Impulsionnelle pour les Systemes de Mesure de l'Elasticite (SEISME)),<br>19 November 2002 (19.11.2002),<br>paragraph [0030]<br>& US 6770033 B1          & WO 2000/055616 A1<br>& FR 2791136 A1          & CN 1343310 A | 1-12 |
| A | JP 2012-196390 A (Fujifilm Corp.),<br>18 October 2012 (18.10.2012),<br>paragraphs [0020] to [0026]; fig. 3<br>(Family: none) | 4-12 |

Form PCT/ISA/210 (continuation of second sheet) (July 2009)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2012100997 A